Europäisches Patentamt

(19) European Patent Office

Office européen des brevets

(11) EP 0 755 303 B1

## (12) FASCICULE DE BREVET EUROPEEN

(45) Date de publication et mention
de la délivrance du brevet:
**10.06.1998 Bulletin 1998/24**

(21) Numéro de dépôt: **95915231.5**

(22) Date de dépôt: **30.03.1995**

(51) Int. Cl.$^6$: **B01J 37/02**, B01J 27/192,
C07C 45/35, C07C 51/25

(86) Numéro de dépôt international:
**PCT/FR95/00407**

(87) Numéro de publication internationale:
**WO 95/26820 (12.10.1995 Gazette 1995/43)**

(54) **COMPOSITIONS CATALYTIQUES DU TYPE ENROBEES, COMPRENANT DU BISMUTH, DU NICKEL, DU COBALT, DU FER ET DU MOLYBDENE, ET LEUR UTILISATION POUR LA PREPARATION D'ALDEHYDES INSATURES**

ZUSAMMENSETZUNGEN DES SCHALENKATALYSATOR-TYPUS, ENTHALTEND WISMUT, NICKEL, KOBALT, EISEN UND MOLYBDÄN, UND DEREN VERWENDUNG ZUR HERSTELLUNG VON UNGESÄTTIGTEN ALDEHYDEN

COATED CATALYTIC COMPOSITIONS INCLUDING BISMUTH, NICKEL, COBALT, IRON AND MOLYBDENUM, AND USE THEREOF FOR PREPARING UNSATURATED ALDEHYDES

(84) Etats contractants désignés:
**AT BE CH DE DK ES FR GB GR IE IT LI LU NL PT SE**

(30) Priorité: **01.04.1994 FR 9403875**

(43) Date de publication de la demande:
**29.01.1997 Bulletin 1997/05**

(73) Titulaire:
**RHONE-POULENC NUTRITION ANIMALE
92160 Antony (FR)**

(72) Inventeur: **PONCEBLANC, Hervé
F-75018 Paris (FR)**

(74) Mandataire: **Le Pennec, Magali
RHONE-POULENC RORER SA,
Direction des Brevets,
20 Avenue Raymond Aron
92165 Antony Cédex (FR)**

(56) Documents cités:
EP-A- 0 017 000          EP-A- 0 068 192
EP-A- 0 319 754          EP-A- 0 493 275
EP-A- 0 551 519          EP-A- 0 573 713
GB-A- 2 052 295

EP 0 755 303 B1

**Description**

La présente invention a trait à des catalyseurs comprenant un support sur lequel est déposée une phase active à base notamment de molybdène, de nickel, de cobalt, de bismuth, de fer, dopée par du phosphore et au moins un élément alcalin.

L'invention concerne de même l'utilisation de telles compositions catalytiques pour l'obtention d'aldéhydes insaturés, par oxydation des oléfines correspondantes.

L'utilisation des compositions catalytiques à base notamment de molybdène, de bismuth et de fer, pour l'obtention d'aldéhydes, par réaction d'oxydation d'une oléfine, est bien connue de l'homme du métier et a fait l'objet de nombreuses recherches.

Différents types de catalyseurs sont donc employés pour effectuer cette réaction. La première catégorie est constituée par des compositions catalytiques se présentant sous une forme massique, mélangée ou non avec un diluant inerte (support). Ces compositions peuvent, entre autres, se trouver sous la forme de grains, d'extrudés ou de pastilles.

Si de tels catalyseurs sont productifs, ils présentent cependant l'inconvénient d'être difficiles à mettre en oeuvre en lit fixe, à l'échelle industrielle. En effet, dans ces conditions, on peut constater une augmentation ponctuelle de la température dans le lit catalytique, pouvant être à l'origine d'un emballement de la réaction.

C'est l'une des raisons du développement de catalyseurs comprenant un support inerte sur lequel une phase catalytiquement active est déposée. Par ce moyen, le contrôle de la température au sein du lit fixe catalytique, et plus précisément de l'homogénéité de cette température, est plus facile.

Néanmoins, ce mode particulier de mise en forme du catalyseur sous entend qu'une part importante dudit catalyseur soit réservée au support. Effectivement, ce dernier représente habituellement plus de la moitié du poids du catalyseur fini. Ainsi, la quantité de phase active entrant dans la composition des catalyseurs enrobés est considérablement réduite par rapport à celle présente dans les catalyseurs massiques dilués. Ceci a pour conséquence directe de baisser de façon importante la productivité des catalyseurs enrobés.

Le document EP 68 192 décrit une composition catalytique constituée d'un support enrobé et d'une phase active. La composition de la phase active est décrite lorsque le catalyseur est utilisé pour la préparation d'acide acrylique, il contient alors Sb, Mo, V, W, Pb ou Ag ou Cu ou Sb ou Ti ou Bl, il ne contient ni P ni alcalins. Pour l'ammoxydation d'hydrocarbures aromatiques le catalyseur contient Sb, V, Fe ou Cu ou Ti ou Co ou Mn ou Ni ou Si. Pour l'oxydation du propylène en acroléine, le catalyseur contient les métaux suivants : Ni, Co, Fe, Bi, Mo. Il peut contenir des alcalins entre 0,05 et 3 % en poids.

La quantité de phase catalytique calculée par rapport au support est comprise en poids entre 10 : 1 et 1 : 1.

Le document EP 17 000 décrit la présence d'alcalins et de phosphore dans un catalyseur contenant les métaux suivants : Mo, Ni, Fe, Bi, Si. Aucune composition catalytique spécifique n'est mentionnée pour l'oxydation du propylène en acroléine alors que le catalyseur utilisé dans l'oxydation de l'acroléine en acide acrylique est spécifiquement décrit.

Plusieurs solutions peuvent être envisagées pour rendre de tels catalyseurs plus attractifs.

La première consisterait à augmenter soit la taille de l'installation industrielle, soit la température de réaction, afin de conserver la capacité de production que l'on aurait en mettant en oeuvre un catalyseur massique. Mais ces moyens posent non seulement des problèmes d'ordre économique mais aussi des problèmes liés directement à la chimie de la réaction et aux performances du catalyseur. En effet, avec une augmentation de la température, la sélectivité en aldéhyde produit diminue et le catalyseur utilisé dans ces conditions se désactive plus rapidement qu'un catalyseur employé à des températures plus faibles.

Une autre solution serait d'augmenter la part de la phase active du catalyseur final, ou en d'autres termes, d'augmenter l'épaisseur de la couche déposée sur le support. Mais dans un tel cas, on se trouverait en présence d'un catalyseur dont la tenue mécanique, et plus particulièrement la résistance à l'attrition, ne serait pas suffisamment importante pour une application à l'échelle industrielle, en lit fixe.

Une autre solution envisageable, et certainement la plus satisfaisante, serait d'ajouter à la phase active des dopants susceptibles d'augmenter la sélectivité en aldéhyde produit, du catalyseur. On a donc proposé d'ajouter à la composition catalytique des additifs comme le phosphore, le potassium. L'usage de tels additifs s'est révélé relativement complexe. En effet, si ces composés ont un rôle certain sur la sélectivité de la réaction en aldéhyde, ils sont la cause d'une baisse des performances du catalyseur, en terme d'activité, si la proportion en ces constituants est trop importante dans la phase active.

La présente invention a pour objet une composition catalytique du type enrobé ne présentant pas les inconvénients précités. Ainsi, cette composition, lorsqu'elle est employée dans des réactions d'oxydation pour l'obtention d'aldéhydes insaturés, permet d'obtenir des résultats améliorés en terme de sélectivité, et par conséquent de rendement en aldéhyde produit, tout en conservant une productivité plus élevée par rapport au catalyseurs enrobés classiques présentant une même teneur en dopants.

Par ailleurs, et ceci constitue un avantage certain, on a constaté que la composition selon l'invention présentait une grande stabilité de performances au cours du temps, donc une durée de vie notablement augmentée.

Ainsi l'invention a pour objet une composition catalytique comprenant un support enrobé d'une phase active à base de bismuth, de molybdène et/ou de tungstène, de cobalt, de nickel, de fer, d'oxygène, dopée par du phosphore et au moins un élément alcalin, ladite phase active représentant 15 à 33 % en poids de la composition catalytique. La phase active entrant dans la composition selon l'invention présente en outre un rapport atomique 100 x (P + éléments alcalins) / Ni compris entre 0,9 et 10.

Le procédé de préparation d'aldéhydes α-β insaturés par oxydation d'oléfines, en phase gazeuse mis en oeuvre en présence de la composition catalytique précitée constitue un second objet de l'invention.

Mais d'autres avantages et caractéristiques de l'invention apparaîtront plus clairement à la lecture de la description et des exemples qui vont suivre.

Ainsi que cela a été indiqué auparavant, la composition catalytique selon l'invention comprend un support sur lequel est déposée une phase active.

La matériau constituant le support est choisi de telle sorte qu'il soit inerte dans les conditions d'utilisation de la composition catalytique. Comme exemples de matériaux pouvant être employés à ce titre, on peut citer la silice, l'alumine, la silice-alumine, l'argile frittée, la terre de diatomée, la magnésie, le silicate de magnésium et des matériaux céramiques comme par exemple la cordiérite, la mullite, la porcelaine, le nitrure de silicium, le carbure de silicium.

Plus particulièrement, le support se présente sous la forme de billes d'un diamètre compris entre 0,5 et 6 mm.

La phase active, élément constitutif de la composition selon l'invention, est à base de bismuth, de molybdène et/ou de tungstène, de cobalt, de nickel, de fer, d'oxygène, dopée par du phosphore et au moins un élément alcalin. Plus particulièrement, l'élément alcalin est le potassium.

La phase active peut comprendre, outre les éléments précités, d'autres éléments constitutifs. Ainsi, il est possible d'inclure de l'arsenic, du bore, ou leur mélange. Il est de même possible de substituer une partie du mélange nickel/cobalt, en conservant toutefois ces deux éléments présents dans la composition, par du manganèse, du magnésium, du plomb, ou leur mélange.

Cependant, selon un mode de réalisation préféré de l'invention, la phase active est à base des éléments cités dans la première liste.

La phase active entrant dans la composition catalytique selon l'invention est en outre caractérisée par le rapport atomique 100 x (P + éléments alcalins) / Ni (dénommé par la suite rapport atomique). Ainsi, la phase active présente un rapport atomique compris entre 0,9 et 10.

Selon un mode de réalisation particulier de l'invention, le rapport atomique est compris entre 0,9 et 7.

Les compositions catalytiques selon l'invention présentent par ailleurs une phase active dans laquelle la quantité de nickel, rapportée à l'ensemble nickel et cobalt, est comprise entre 10 et 60 % en moles.

Il a été trouvé d'une façon tout à fait surprenante que la composition catalytique contenant du nickel, telle que définie auparavant, pouvait contenir une quantité beaucoup plus importante en éléments dopants (le phosphore et les éléments alcalins) que les compositions exemptes de nickel, sans que l'on constate par ailleurs d'effet rédhibitoire sur l'activité des compositions catalytiques résultantes.

Par conséquent, les compositions selon l'invention, lorsqu'elles sont mises en oeuvre dans des réactions d'oxydation d'oléfines, sont particulièrement sélectives en aldéhyde et présentent une productivité acceptable.

Selon un mode de réalisation particulier, le rapport atomique de l'élément alcalin sur le phosphore est compris entre 0,3 et 3 inclus.

De préférence, ce rapport est compris entre 0.5 et 2 inclus.

Comme indiqué précédemment, la composition catalytique comprend une phase active déposée sur un support. La quantité de phase active représente 15 à 33 % en poids de la composition catalytique. De préférence, celle-ci représente 20 à 30 % en poids de la composition catalytique.

La phase active peut être obtenue par tout moyen classique.

On peut envisager par exemple de fabriquer ladite phase active par mélange des oxydes des éléments constitutifs de la phase active ou de composés susceptibles de se transformer en oxyde lorsqu'ils sont portés à des températures élevées. Le mélange est ensuite soumis à une étape de calcination, éventuellement suivie et/ou précédée par un broyage dudit mélange (technique de chamottage).

Une autre méthode convenable pour la préparation de la phase active consiste à préparer un mélange à base des éléments constitutifs de la phase active, d'effectuer une opération de séchage puis de calcination du produit résultant.

Les éléments constitutifs mis en oeuvre selon cette méthode, peuvent être utilisés notamment sous forme d'oxydes, ou de précurseurs d'oxydes tels que les sels d'acides inorganiques ou organiques. A titre d'exemple, on peut citer les nitrates, les sulfates, les halogénures, les sels d'ammonium, le formiate, l'oxalate, le tartrate, l'acétate. Conviennent aussi les dérivés d'oxydes comme notamment les hydroxydes, les oxyhalogénures, les alkoxydes, les glycoxydes.

Lesdits sels sont ensuite mis en suspension ou en solution, dans tout solvant ou dispersant approprié, et de préférence dans l'eau.

L'opération de séchage, effectuée plus particulièrement sous une atmosphère non réductrice, peut être réalisée par évaporation jusqu'à siccité ou par atomisation.

Selon la première variante, le séchage est effectué en deux temps : le premier consistant à évaporer le solvant ou dispersant du mélange, jusqu'à siccité, le second à sécher la pâte ainsi obtenue. Généralement, la première étape est réalisée sous agitation à une température variant de 20 à 100°C pendant la durée nécessaire pour obtenir une pâte non coulante. La pâte résultante est séchée, dans un second temps, sous une atmosphère de préférence non réductrice, comme l'oxygène ou l'air par exemple. Cette dernière opération est effectuée sur une durée moyenne de 15 heures à une température de séchage d'environ 120°C.

Selon la seconde variante, le séchage est effectué en atomisant la solution ou suspension, par tout moyen connu de l'homme du métier, comme par exemple les atomiseurs de type "flash" tels que ceux revendiqués dans les demandes de brevets français publiées sous les numéros suivants : 2 257 326, 2 419 754, 2 431 321, ou encore de type BUCHI. La température d'atomisation est généralement de l'ordre de 150 à 300°C et l'atmosphère sous laquelle elle est effectuée est de préférence non réductrice, comme précédemment indiqué.

Le produit séché est ensuite soumis à une étape de calcination sous une atmosphère non réductrice à une température appropriée pour la formation d'oxydes. Elle est habituellement comprise entre 200 et 1200°C.

Préalablement à l'étape de calcination, le produit séché peut subir une étape de broyage. Il est à noter que le produit calciné peut éventuellement subir aussi un tel traitement.

Un troisième mode convenable pour la préparation de la phase active consiste à effectuer une ou plusieurs étapes successives de précipitation des éléments constitutifs présents sous forme soluble. Cette étape est réalisée préalablement au séchage, les autres étapes mentionnées dans la variante précédente étant par ailleurs conservées.

Les composés susceptibles d'être utilisés comme agent précipitant sont choisis parmi ceux qui se combinent à un ou plusieurs des éléments constitutifs, sous la forme d'un composé insoluble dans le milieu, ledit composé insoluble étant un précurseur d'oxyde. Classiquement, mais sans y être limité, l'ammoniaque, l'acide chlorhydrique, l'acide citrique conviennent.

Habituellement, la précipitation est mise en oeuvre sous agitation.

La température à laquelle elle est réalisée, varie généralement entre 20°C et la température d'ébullition du solvant choisi.

Enfin, la ou les étapes de précipitation peuvent si nécessaire être suivies d'étapes de mûrissement. Celles-ci consistent à laisser le précipité en suspension, sous agitation, pendant une durée variant de 30 minutes à 4 heures environ.

Selon un mode de réalisation préféré du procédé de préparation de la composition catalytique selon l'invention, on prépare tout d'abord une phase active exempte de dopants, c'est-à-dire exempte de phosphore et d'éléments alcalins ; ceux-ci n'étant introduits qu'au moment du dépôt de la phase active sur le support (enrobage).

L'enrobage est l'opération par laquelle on entoure progressivement des billes de support d'une couche externe de phase active.

Cette opération est réalisée de manière en soi connue par introduction desdites billes dans un drageoir tournant. Celui-ci est muni de moyens pour introduire des particules de phase active, dont la granulométrie est de préférence inférieure à 400 $\mu$m, et de moyens pour introduire une solution aqueuse d'agent d'adhésion. Comme mentionné auparavant, et selon une caractéristique avantageuse du présent procédé, l'introduction des dopants est donc réalisée lors de cette étape.

Plus particulièrement, on introduit dans le drageoir au moins un composé d'un élément alcalin, de préférence le potassium, et au moins un composé du phosphore sous forme de solution, plus particulièrement aqueuse, simultanément avec la composition catalytique exempte desdits dopants et une solution d'un agent d'adhésion. De préférence, la solubilité dans l'eau du ou des composés du phosphore et celle du ou des composés de l'élément alcalin mesurée à 25°C, est supérieure à 10 g/l.

Lesdits composés sont choisis parmi ceux qui n'interfèrent pas ou sont inertes vis-à-vis des autres composants de la solution d'enrobage, notamment l'agent d'adhésion et le cas échéant, d'autre additifs comme par exemple un agent porogène. Ces composés peuvent en outre être décomposables par la chaleur lors de la calcination ultérieure.

En pratique, l'addition des composés choisis du phosphore et de l'élément alcalin, et notamment du potassium, doit conduire la solution aqueuse à une valeur de pH comprise entre 3 et 11.

Selon une première variante, on emploie des composés comprenant l'un ou l'autre des dopants précités. Ainsi, en tant que composé apportant l'élément alcalin, on peut mentionner la potasse, la soude. En ce qui concerne les composés à base de phosphore, on peut citer sans intention de se imiter, les phosphates minéraux ou organiques, l'acide phosphorique.

Selon une seconde variante, préférée, de la présente invention, on utilise au moins un composé à base des deux types de dopants, comme par exemple, le dihydrogénophosphate de potassium, de sodium, le monohydrogénophosphate de potassium, de sodium.

Il est à noter que la combinaison des deux variantes précédentes est tout à fait envisageable. Ceci est particulièrement indiqué, lorsque, en mettant en oeuvre la seconde variante, un complément en l'un et/ou l'autre des dopants est nécessaire.

Selon un mode de réalisation particulier, les composés du phosphore et de l'élément alcalin sont introduits dans la solution aqueuse d'agent d'adhésion.

L'opération d'enrobage est conduite dans un drageoir tournant faisant entre 10 et 20 tours par minute dans lequel on place la quantité requise de billes de support rugueuses et dépoussiérées, à recouvrir de phase active. On introduit alors simultanément par une goulotte, la composition catalytique intermédiaire broyée, et la solution aqueuse d'agent d'adhésion et du (des) composé (s) de phosphore et d'élément alcalin.

Dans une variante préférée, les billes ont été préalablement humectées par une solution aqueuse d'agent d'adhésion ne contenant pas de dopant.

L'opération est poursuivie jusqu'à ce que toute la solution d'enrobage, puis toute la phase active soient utilisées. La rotation est maintenue quelques minutes pour bien tasser la couche de phase active sur les billes.

Puis les billes sont séchées par de l'air chaud, entre 80 et 150°C pendant 10 à 30 minutes et calcinées.

Selon un mode de réalisation particulier, l'étape de calcination est effectuée en deux périodes. La première consiste à porter, en 3 à 15 heures, la température entre 450 et 500°C. En second lieu, on refroidit le mélange en 3 à 10 heures.

Dans une variante préférée, une deuxième calcination est réalisée successivement et dans les mêmes conditions de variation de température que la première calcination.

Dans une autre variante préférée, la température de calcination est de 480°C.

La présente invention concerne enfin un procédé de préparation d'aldéhydes $\alpha$-$\beta$ insaturés par oxydation, en phase gazeuse, des oléfines correspondantes, la réaction étant mise en oeuvre en présence de la composition catalytique selon l'invention.

Plus particulièrement, l'invention est appropriée pour l'obtention d'acroléine ou de méthacroléine par oxydation du propylène ou de l'isobutylène respectivement.

La réaction consiste à introduire dans un réacteur, l'oléfine, une source d'oxygène et éventuellement de l'eau.

L'oxygène pur ou dilué dans des gaz inertes tels que l'azote, les oxydes de carbone et/ou les gaz rares, peut être utilisé en tant que source d'oxygène. Il est à noter que de tels gaz peuvent provenir d'un recyclage des gaz dans le procédé.

D'une façon avantageuse, la source d'oxygène employée est l'air.

La quantité d'oléfine dans le flux d'alimentation est habituellement comprise entre 6 et 10 % exprimée en mole.

La quantité d'oxygène présent dans le flux d'alimentation est en général comprise entre 10 et 15 % exprimée en mole.

La quantité de vapeur d'eau introduite est habituellement comprise entre 0 et 40 % exprimée en mole. De préférence, la quantité d'eau est comprise entre 20 et 40 % en mole.

Les gaz inertes précités représentent le complément à 100 %.

Généralement, la température de réaction se situe aux alentours de 100 à 450°C.

La pression des réactifs à l'entrée du réacteur est comprise entre 1 et 5 bar. Plus particulièrement la pression est comprise entre 1 et 3 bar.

De préférence, la pression est légèrement supérieure à la pression atmosphérique pour tenir compte des pertes de charge dans l'installation.

En règle générale, la présente réaction est effectuée en présence d'un lit fixe de catalyseur, bien que l'emploi de lits fluidisés soit envisageable.

Des exemples concrets mais non limitatifs de l'invention vont maintenant être présentés.

**EXEMPLE 1**

Préparation d'un catalyseur de formule générale

$$Co_{6,1} Ni_{3,8} Fe_1 Bi_1 Mo_{12} P_{0,05} K_{0,1} O_\alpha$$

1 - Préparation de la phase active exempte de dopant.

On prépare les solutions suivantes :

**A** :

167,6 g d'heptamolyodate d'ammonium [$(NH_4)_6 Mo_7O_{24}, 4H_2O$]
760 ml d'eau déminéralisée

Le mélange est réalisé à 80°C, sous agitation.

**B** :

(a) 32,1 g de nitrate de fer [Fe(NO$_3$)$_3$, 9H$_2$O] 25 ml d'eau déminéralisée

(b) 38,4 g de nitrate de bismuth [Bi(NO$_3$)$_3$, 5H$_2$O] 4 ml d'acide nitrique pur (d= 1,33) puis dilution avec 28,5 ml d'eau, ajoutée goutte à goutte, sous agitation

On introduit la solution (a), goutte à goutte, dans la solution (b).

**C** :

(a) 138g de nitrate de cobalt [Co(No$_3$)$_2$, 6H$_2$O] 90 ml d'eau.

(b) 91,9g de nitrate de nickel [Ni(No$_3$)$_2$ 6H$_2$O] 10 ml d'eau

On mélange ensuite les solutions (a) et (b).

On introduit lentement, sous agitation, la solution **B** dans la solution **C**.

Le mélange résultant est introduit en 30 minutes dans la solution **A**, sous agitation forte. Le pH du milieu est de 2,05 en fin d'addition et l'on obtient une suspension de couleur rose.

150 ml d'ammoniaque (d = 0,92) sont ajoutés à la suspension en 20 minutes, sous agitation. Le pH en fin d'introduction est de 7,1 et l'on obtient une suspension marronbeige.

La suspension est alors chauffée à 60°C pendant 4 heures, sous agitation.

Le milieu est ensuite laissé pendant 2 heures, sans chauffage ni agitation.

Le solide est filtré et lavé en utilisant une quantité d'eau déminéralisée totale de 1 litre.

Le solide est ensuite séché 16 heures à l'étuve à 120°C, puis calciné 6 heures à 400°C sous air et broyé.

2 - <u>Préparation de la composition catalytique enrobée.</u>

L'enrobage s'effectue dans un drageoir de 20 cm de diamètre et contenant 100 g de billes d'argile frittée à 1100°C, de rugosité de surface 0,3 et de diamètre 4,8 mm. Les billes sont préalablement mouillées par une première solution de glucose à 0,1 g/l pulvérisée à un débit de 2,5 à 3,5 ml/mn.

Quand toutes les billes sont mouillées, une goulotte permet d'introduire 44 g de phase active exempte de dopants, en 30 minutes environ. Pendant l'introduction de la poudre, le drageoir continue de tourner et 10 ml d'une seconde solution de glucose à 0,1 g/l contenant du K$_2$HPO$_4$ à 13,56 g/l est pulvérisée sur les billes.

Une fois l'ensemble de la solution et de la phase active exempte de dopants introduit, la rotation est maintenue environ quelques minutes. On sèche ensuite sous un courant d'air chaud pendant 15 minutes.

Les billes ainsi enrobées et séchées sont introduites dans des étuves ventilées afin d'être calcinées.

Le profil en température est le suivant :

- montée à 240 °C en 6 heures, puis à 480°C en 8 heures;
- palier de 6 heures à 480 °C;
- refroidissement à 150°C en 10 heures;
- montée à 480 °C en 10 heures;
- palier de 6 heures à 480 °C;
- refroidissement final en 10 heures jusqu'à température ambiante.

On retire donc de l'étuve 143,5 g de catalyseur final sur lequel le taux massique de phase active est de 30 %.

**EXEMPLE 2**

Préparation d'un catalyseur de formule générale

$$Co_{6,1}\ Ni_{3,8}\ Fe_1\ Bi_1\ Mo_{12}\ P_{0,075}K_{0,15}\ O_\alpha$$

1 - <u>Préparation de la phase active exempte de dopants.</u>

On procède comme dans l'exemple 1.

2 - <u>Préparation de la composition catalytique enrobée.</u>

On procède comme dans l'exemple précédent excepté le fait que la seconde solution de glucose (10 ml) contient 20,32 g/l de $K_2HPO_4$.

**EXEMPLE 3 Comparatif**

Préparation d'un catalyseur de formule générale :

$$Co_{9,6} \ Fe_1 \ Bi_1 \ Mo_{12} \ P_{0,0125} \ K_{0,025} \ O_\alpha$$

1 - <u>Préparation de la phase active exempte de dopants.</u>

On opère comme dans l'exemple 1 excepté le fait que l'on n'ajoute pas de nitrate de nickel (solution **C**) et que l'on utilise, à la place, 230,2 g de nitrate de cobalt.

2 - <u>Préparation de la composition catalytique.</u>

On procède comme dans l'exemple 1 mais la seconde solution de glucose comprend 3,42 g/l de $K_2HPO_4$.

**EXEMPLE 4**

Préparation d'un catalyseur de formule générale

$$Co_9 \ Ni_1 \ Fe_1 \ Bi_1 \ Mo_{12}O_\alpha$$

1 - <u>Préparation de la phase active exempte de dopants.</u>

On procède comme dans l'exemple 1 excepté le fait que la solution **C** est obtenue par mélange d'une solution comprenant 207,5 g de nitrate de cobalt dans 90 ml d'eau et d'une solution comprenant 23 g de nitrate de nickel dans 10 ml d'eau..

2 - <u>Préparation de la composition catalytique.</u>

On procède comme dans l'exemple 1 mais la seconde solution de glucose ne comprend pas de dopants.
Par ailleurs, on opère de telle sorte que le catalyseur résultant comprenne 18 % en poids de phase active.

**EXEMPLE 5**

Préparation d'un catalyseur de formule générale

$$Co_{6,1} \ Ni_{3,8} \ Fe_1 \ Bi_1 \ Mo_{12}O_\alpha$$

1 - <u>Préparation de la phase active exempte de dopants.</u>

On procède comme dans l'exemple 1.

2 - <u>Préparation de la composition catalytique.</u>

On procède comme dans l'exemple 1 mais la seconde solution de glucose ne comprend pas de dopant.
Par ailleurs, on opère de telle sorte que le catalyseur résultant comprenne 18 % en poids de phase active.

**EXEMPLE 6 Comparatif**

Préparation d'un catalyseur de formule générale

$$Co_{9,6} \ Fe_1 \ Bi_1 \ Mo_{12}O_\alpha$$

1 - <u>Préparation de la phase active exempte de dopants.</u>

On procède comme dans l'exemple 1 excepté le fait que la solution **C** comprend 230,2 g de nitrate de cobalt dans 90 ml d'eau.

2 - <u>Préparation de la composition catalytique.</u>

On procède comme dans l'exemple 1 mais la seconde solution de glucose ne comprend pas de dopant.
Par ailleurs, on opère de telle sorte que le catalyseur résultant comprenne 18 % en poids de phase active.

**EXEMPLE 7**

Les performances des catalyseurs décrits dans les exemples 1, 2 et 3 comparatif, ont été déterminées dans les conditions suivantes :
90 ml de catalyseur, enrobé, non dilué, sont contenus dans un réacteur de 80 cm de haut et 15,5 mm de diamètre interne.
Les gaz d'entrée du réacteur sont constitués d'un mélange de propylène, d'air et d'eau, dans les proportions molaires (exprimées en %) : 7 / 57 / 36.
La pression dans le réacteur est maintenue à 1,8 bar absolus.
La vitesse volumique horaire, définie par le rapport débit gazeux total (Nl/h) du volume de catalyseur, est de 1265 $h^{-1}$, soit une charge de propylène de 166 g par heure et par litre de catalyseur.
Le taux de conversion du propylène $X_{C3H6}$ est défini par le rapport suivant :

$$\frac{\text{nombre de moles de propylène converti}}{\text{nombre de moles de propylène à l'entrée}} \times 100$$

La sélectivité en produit i, Si, est définie comme le rapport suivant :

$$\frac{\text{Nombre de moles de produit i formé}}{\text{Nombre de moles de propylène converti}} \times 100$$

Le rendement en produit i, Ri, est défini comme le produit de $X_{C3H6}$ et Si.
Les performances catalytiques des catalyseurs sont mesurées après 30 heures sous réactifs.

TABLEAU 1

| EXEMPLE | $T_{bain}$ (°C) | $X_{C3H6}$ (%) | $S_{acro}$ (%) | $S_{acry}$ (%) | $S_{COx}$ (%) | $R_{acro}$ (%) |
|---|---|---|---|---|---|---|
| Exemple 1 | 330 | 89,5 | 87,7 | 7,2 | 1,9 | 78,5 |
| Exemple 2 | 330 | 82,7 | 90,9 | 4,5 | 1,5 | 75,2 |
| Exemple 3 Comparatif | 330 | 94,5 | 78,0 | 11,0 | 5,2 | 73,7 |
| acro = acroléine<br>acry = acide acrylique<br>COx = produits de combustion | | | | | | |

**EXEMPLE 8**

Test de stabilité en fonction du temps.

La stabilité de performances catalytiques des catalyseurs décrits dans les exemples 2 et 3 comparatif, a été déterminée dans les conditions suivantes :
2 litres de catalyseur, enrobé, non dilué, sont contenus dans un réacteur de 26 mm de diamètre interne et de 4 mètres de hauteur.
Les gaz d'entrée du réacteur sont constitués d'un mélange de propylène, d'air et d'eau, dans les proportions molaires (exprimées en %) : 8,9 / 62,5 / 28,6.

La pression dans le réacteur est maintenue à 1,8 bar absolus.

La vitesse volumique horaire définie comme dans l'exemple 3, est de 1370h$^{-1}$, soit une charge de propylène de 230 g par heure et par litre de catalyseur.

Les essais sont menés en maintenant la conversion du propylène à une valeur constante, égale à 90 % environ, en augmentant progressivement la température de bain.

Le tableau 2 permet de comparer les niveaux de température de bain nécessaires pour maintenir constante la conversion, et les performances catalytiques des catalyseurs des exemples 2 et 3 comparatif, pendant des essais d'environ 2000 heures.

Les taux de conversion du propylène $X_{C3H6}$, sélectivités Si et rendement Ri en les différents produits i sont définis comme dans l'exemple précédent.

TABLEAU 2

| EXEMPLE | Formule catalytique | temps (heures) | $T_{bain}$ (°C) | $X_{C3H6}$ (%) | $S_{acro}$ (%) | $R_{acro}$ (%) |
|---|---|---|---|---|---|---|
| Exemple 2 | $Co_{6,1} Ni_{3,8} Fe_1 Bi_1 Mo_{12} P_{0,075} K_{0,15} O_\alpha$ | 800 | 328 | 89,0 | 92,2 | 82,1 |
| | | 1400 | 327 | 90,0 | 92,2 | 83 |
| | | 1600 | 328 | 90,2 | 91,9 | 82,9 |
| | | 1800 | 327,5 | 90,8 | 92,5 | 84,0 |
| Exemple 3 comparatif | $Co_{9,6} Fe_1 Bi_1 Mo_{12} P_{0,0125} K_{0,025} O_\alpha$ | 1400 | 343,5 | 89,5 | 86,0 | 77,0 |
| | | 1600 | 345,5 | 89,9 | 85,2 | 76,6 |
| | | 1800 | 347 | 91,3 | 84,0 | 76,7 |
| | | 2000 | 352 | 90,7 | 83,0 | 75,3 |

Ce tableau indique clairement que les catalyseurs selon l'invention présentent une stabilité très nettement supérieure à ceux de l'art antérieur puisqu'après 1800 heures, la température de bain est identique à la température initiale.

Par contre, elle a été augmentée de près de 10°C dans le cas des catalyseurs de l'art antérieur.

**EXEMPLE 9**

Test de vieillissement accéléré

Les catalyseurs exempts de dopants obtenus dans les exemples 4 et 5 et 6 comparatif ont été testés dans les conditions suivantes :

Un lit de 90 ml constitué de catalyseur enrobé, dilué au quart en volume avec des billes d'argile non enrobées, est contenu dans le réacteur défini dans l'exemple 7.

Les gaz d'entrée du réacteur sont constitués d'un mélange de propylène, d'air et d'eau dans les proportions molaires (exprimées en %) : 7 / 57 / 36.

La pression dans le réacteur est maintenue à 1,8 bar absolu.

La vitesse volumique horaire est de 4285 h$^{-1}$, soit une charge de propylène de 562g/h/l de catalyseur actif.

L'essai de vieillissement est réalisé en augmentant au cours du temps la température de bain (entre 400 et 420 °C).

Les résultats de ce test catalytique sont rassemblés dans le tableau 3 ci-dessous, dans lequel on a indiqué le nombre de point de conversion (Xg) perdu en 100 heures de test.

| EXEMPLE | Formule catalytique | Δconversion |
|---|---|---|
| Exemple 4 | $Co_9 Ni_1 Fe_1 Bi_1 Mo_{12} O_\alpha$ | 8 |
| Exemple 5 | $Co_{6,1} Ni_{3,8} Fe_1 Bi_1 Mo_{12} O_\alpha$ | 4 |

(suite)

| EXEMPLE | Formule catalytique | Δconversion |
|---|---|---|
| Exemple 6 Comparatif | $Co_{9,6} Fe_1 Bi_1 Mo_{12}0_\alpha$ | 12 |

Ce tableau montre que les catalyseurs comprenant du nickel présentent une activité catalytique plus stable dans le temps que les catalyseurs qui en sont dépourvus, et il semble en outre que cet effet soit d'autant plus marqué que la teneur en nickel dans la phase active est importante.

**Revendications**

1. Composition catalytique comprenant un support enrobé d'une phase active à base de bismuth, de molybdène et/ou de tungstène, de cobalt, de nickel, de fer, d'oxygène, dopée par du phosphore et au moins un élément alcalin, caractérisée en ce que ladite phase active représentant 15 à 33 % en poids de la composition catalytique et la phase active présente un rapport atomique 100 x (P + éléments alcalins) / Ni compris entre 0,9 et 10.

2. Composition selon la revendication précédente, caractérisée en ce que le rapport atomique est compris entre 0,9 et 7.

3. Composition catalytique selon l'une quelconque des revendications précédentes, caractérisée en ce que le rapport atomique de l'élément alcalin sur le phosphore est compris entre 0,3 et 3 inclus.

4. Composition selon l'une quelconque des revendications précédentes, caractérisée en ce que la quantité de nickel présente dans la phase active, rapportée à l'ensemble nickel et cobalt, est comprise entre 10 et 60 % en poids.

5. Procédé de préparation d'une composition catalytique selon la revendication 1 caractérisé en ce que :

   - dans une première étape, on humidifie le support avec une solution d'agent adhérant,
   - dans une deuxième étape, on prépare une solution catalytique ou une suspension contenant différents oxydes ou leur précurseurs, libre de dopants, puis on la sèche et la calcine,
   - dans une troisième étape, on enrobe le support avec le catalyseur préparé dans la deuxième étape, et avec une solution d'agent adhésif et des dopants,
   - dans une quatrième étape, on calcine la composition obtenue dans la troisième étape.

6. Procédé de préparation d'aldéhydes $\alpha$-$\beta$ insaturés par oxydation d'oléfines, en phase gazeuse, caractérisée en ce qu'il est réalisé en présence d'une composition catalytique selon l'une quelconque des revendications 1 à 4.

7. Procédé de préparation d'acroléine par oxydation du propylène, en phase gazeuse, caractérisé en ce qu'il est réalisé en présence de la composition catalytique selon l'une quelconque des revendications 1 à 4.

**Claims**

1. Catalytic composition comprising a support coated with an active phase which is based on bismuth, molybdenum and/or tungsten, cobalt, nickel, iron, oxygen and is doped with phosphorus and at least one alkali metal element, characterized in that the said active phase represents from 15 to 33 % by weight of the catalytic composition, and the active phase has an atomic ratio 100 X (P + alkali metal elements) /Ni of between 0.9 and 10.

2. Composition according to the preceding claim, characterized in that the atomic ratio is between 0.9 and 7.

3. Catalytic composition according to any one of the preceding claims, characterized in that the atomic ratio of the alkali metal element to the phosphorus is from 0.3 to 3 inclusive.

4. Composition according to any one of the preceding claims, characterized in that the quantity of nickel present in the active phase, relative to nickel and cobalt together, is between 10 and 60 % by weight.

5. Process for the preparation of a catalytic composition according to claim 1, characterized in that:

   - in a first step, the support is wetted with a solution of adhesion agent,

- in a second step, a catalytic solution or a suspension is prepared containing various oxides or their precursors, which is free from dopants, and then this solution or suspension is dried and calcined,
- in a third step, the support is coated with the catalyst prepared in the second step, and with a solution of adhesion agent and of the dopants, and
- in a fourth step, the composition obtained in the third step is calcined.

6. Process for the preparation of $\alpha,\beta$-unsaturated aldehydes by oxidation of olefins in the gas phase, characterized in that it is carried out in the presence of a catalytic composition according to any one of claims 1 to 4.

7. Process for the preparation of acrolein by oxidation of propylene in the gas phase, characterized in that it is carried out in the presence of the catalytic composition according to any one of claims 1 to 4.

**Patentansprüche**

1. Katalytische Zusammensetzung, die einen Träger umfaßt, der mit einer aktiven Phase auf der Basis von Bismut, Molybdän und/oder Wolfram, Kobalt, Nickel, Eisen Sauerstoff, die mit Phospor und wenigstens einem Alkalielement dotiert ist, umhüllt ist, dadurch gekennzeichnet, daß besagte aktive Phase 15 bis 33 Gew.-% der katalytischen Zusammensetzung darstellt und die aktive Phase ein Atomverhältnis 100 x (P + Alkalielemente) / Ni zwischen 0,9 und 10 aufweist.

2. Zusammensetzung gemäß dem vorhergehenden Anspruch, dadurch gekennzeichnet, daß das Atomverhältnis zwischen 0,9 und 7 liegt.

3. Katalytische Zusammensetzung gemäß einem der vorhergehenden Ansprüche, dadurch gekennzeichnet, daß das Atomverhältnis des Alkalielements zu Phosphor zwischen einschließlich 0,3 und 3 liegt.

4. Zusammensetzung gemäß einem der vorhergehenden Ansprüche, dadurch gekennzeichnet, daß die in der aktiven Phase vorhandene Nickelmenge, bezogen auf die Summe Nickel und Kobalt, zwischen 10 und 60 Gew.-% liegt.

5. Verfahren zur Herstellung einer katalytischen Zusammensetzung gemäß Anspruch 1, dadurch gekennzeichnet, daß:

- man in einem ersten Schritt den Träger mit einer Haftmittellösung anfeuchtet,
- man in einem zweiten Schritt eine dotierungsmittelfreie katalytische Lösung oder eine Suspension herstellt, die verschiedene Oxide oder ihre Vorstufen enthält, man sie dann trocknet und glüht,
- man in einem dritten Schritt den Träger mit dem im zweiten Schritt hergestellten Katalysator und mit einer Lösung des Haftmittels und der Dotierungsmittel umhüllt,
- man in einem vierten Schritt die im dritten Schritt erhaltene Zusammensetzung calciniert.

6. Verfahren zur Herstellung von $\alpha,\beta$-ungesättigten Aldehyden durch Oxidation von Olefinen in der Gasphase, dadurch gekennzeichnet, daß es in Gegenwart einer katalytischen Zusammensetzung gemäß einem der Ansprüche 1 bis 4 durchgeführt wird.

7. Verfahren zur Herstellung von Acrolein durch Oxidation von Propylen in der Gasphase, dadurch gekennzeichnet, daß es in Gegenwart der katalytischen Zusammensetzung gemäß einem der Ansprüche 1 bis 4 durchgeführt wird.